# EUROPÄISCHE PATENTANMELDUNG

(11) **EP 1 125 568 A2**
(43) Veröffentlichungstag der Anmeldung: **22.08.2001**
(21) Anmeldenummer: 01810106.3
(22) Anmeldetag: 02.02.2001
(51) Int. Cl.: A61F 9/007

(54) **Verfahren und Vorrichtung zum Verbesseren sowie zur Aufrechterhaltung des Kammerwasserablusses in einem Auge**

(30) Priorität: 15.02.2000 US 503884
(71) Anmelder: GRIESHABER & CO. AG SCHAFFHAUSEN, CH-8203 Schaffhausen (CH)
(72) Erfinder: Grieshaber, Hans R., 8200 Schaffhausen (CH); Stegmann, Robert, Prof.M.D., Pretoria 0181 (ZA)
(74) Vertreter: Althoff, Gerhard

(57) **Zusammenfassung**

Die Erfindung bezieht sich auf ein Verfahren zum Verbessern des Kammerwasserabflusses in einem Auge sowie auf eine Vorrichtung zur Aufrechterhaltung des verbesserten Kammerwasserabflusses.

Gemäss dem Verfahren wird durch einen ersten und zweiten Einschnitt (Inzision) an der Oberfläche der Sklera (3) und durch Aufklappen analog ausgebildeter erster und zweiter Skleralappen (10;12) der Schlemmsche Kanal (5) freigelegt und dieser vorzugsweise durch Injizierung eines hochviskosen Mediums gedehnt. Zur Bildung einer spaltförmigen Öffnung (21) wird der zweite Skleralappen (12) oder ein Teilstück (12.1) desselben im Bereich der Schwalbeschen Linie (7) von der teilweise kammerwasserdurchlässigen Descemet-Membran (6) gelöst und das abgelöste Teilstück (12.1) mittels mehrerer Auflageteile eines in dem freigelegten Teilstück (18) des Schlemmschen Kanals (5) angeordneten Stützelements (40) in Offenstellung gehalten.

Zur Aufrechterhaltung des verbesserten Kammerwasserabflusses kann zu beiden Seiten des freigelegten Teilstücks (18) in das Lumen (19) mindestens ein von dem Gewebe und/oder von dem Kammerwasser abbaubares Stützelement (30) und zudem zur Beibehaltung der Öffnung (21) sowie Auflage des heruntergeklappten ersten Skleralappens (10) in dem freigelegten Teilstück (18) das Stützelement (40) implantiert werden.

## Beschreibung

Die Erfindung bezieht sich auf ein Verfahren zum Verbessern des Kammerwasserabflusses in einem Auge sowie auf eine Vorrichtung zur Aufrechterhaltung des Kammerwasserabflusses.

Aus der US-A 5,360,399 ist ein Verfahren bekannt, mittels welchem das dem Schlemmschen Kanal vorgelagerte und infolge krankhafter Veränderungen den Abfluss des Kammerwassers teilweise oder vollständig obstruierende Trabekulargewebe mittels einer in den Schlemmschen Kanal injizierten hochviskosen Lösung etwa hydraulisch gedehnt und infolge dessen das Trabekulargewebe an einigen Stellen für den erforderlichen Abfluss des Kammerwassers geöffnet wird.

Aus der EP-A 0 898 947 ist es weiterhin bekannt, in das Lumen des Schlemmschen Kanals ein Stützelement aus biokompatiblem Material, beispielsweise aus nichtrostendem Edelmetall oder geeignetem Kunststoff zu implantieren, wobei mit dem Stützelement der Schlemmsche Kanal in gedehnter Stellung gehalten und infolge dessen der Abfluss des Kammerwassers über das natürliche Kanalsystem erreicht wird.

Der Erfindung liegt die Aufgabe zugrunde ein Verfahren anzugeben sowie eine gattungsgemässe Vorrichtung zu schaffen, mittels welchem/welcher eine den Druck in einem Auge regulierende Zirkulation des Kammerwassers sowie der erforderliche Abfluss desselben über das natürliche Kanalsystem verbessert und aufrechterhalten wird.

Das Verfahren gemäss der Erfindung ist gekennzeichnet durch einen ersten lamellaren Einschnitt an der Oberfläche der Sklera mit in Richtung der Kornea aufgeschwenktem ersten Skleralappen sowie analog ausgebildeter erster Ausnehmung, einen innerhalb der ersten Ausnehmung vorgesehenen zweiten Einschnitt mit aufgeschwenktem zweiten Skleralappen und analog ausgebildeter zweiter Ausnehmung sowie ein in diesem Bereich freigelegtes Teilstück des Schlemmschen Kanals, wobei zu beiden Seiten der zweiten Ausnehmung in das Lumen und wahlweise in das freigelegte Teilstück jeweils mindestens ein von dem Gewebe des Schlemmschen Kanals und/oder vom Kammerwasser abbaubares Stützelement implantiert und nach dem Abtrennen des zweiten Skleralappens der erste Skleralappen auf eine analog dem zweiten Einschnitt ausgebildete Auflagefläche gelegt und der dadurch gebildete subsklerale Raum vor dem vollständigen Verschliessen mit viskosem Medium gefüllt wird.

Nach einem weiteren Merkmal des Verfahrens besteht zudem die Möglichkeit, dass durch eine geringe Anpresskraft im Bereich der Schwalbeschen Linie der zweite Skleralappen von der teilweise kammerwasserdurchlässigen Descemet-Membran gelöst wird und dadurch eine spaltförmige Öffnung (window) entsteht. Mit einem implantierten Stützelement wird das gelöste Teilstück des abgetrennten zweiten Skleralappens derart gehalten, dass die Vorderkammer im Bereich des Kammerwinkels über die Descemet-Membran und der spaltförmigen Öffnung mit dem subskleralen Raum in Verbindung steht.

Hierdurch wird erreicht, dass das einerseits über das Trabekulargewebe auf natürlichem Weg in den Schlemmschen Kanal abgeleitete Kammerwasser zusätzlich noch von der Vorderkammer durch die teilweise kammerwasserdurchlässige Descemet-Membran und durch die spaltförmige Öffnung in den mit dem Schlemmschen Kanal in Verbindung stehenden subskleralen Raum der Sklera abgeleitet wird.

Die Vorrichtung gemäss dem Oberbegriff des Anspruchs 9 ist gekennzeichnet durch mindestens ein ringförmig, kugelförmig oder röhrchenförmig ausgebildetes und im Lumen des Schlemmschen Kanals angeordnetes Stützelement, welches aus abbaubarem Material, insbesondere aus einem von dem Gewebe des Schlemmschen Kanals und/oder von dem Kammerwasser abbaubarem Material hergestellt ist.

Weitere Vorteile, Merkmale und Einzelheiten der Erfindung ergeben sich aus den einzelnen Patentansprüchen und der nachstehenden Beschreibung sowie der Zeichnung.

Die Erfindung wird nachstehend anhand von Ausführungsbeispielen in Verbindung mit der Zeichnung beschrieben. Es zeigt:
- **Fig.1**: ein in schematischer Ansicht und in grösserem Massstab dargestelltes Teilstück eines Auges mit einem ersten parabolischen Einschnitt in der Sklera und aufgeklapptem ersten Skleralappen;
- **Fig.2**: das entlang der Linie II-II im Schnitt dargestellte Teilstück des Auges gemäss Fig.1 mit aufgeklapptem ersten Skleralappen;
- **Fig.3**: das in Ansicht dargestellte Teilstück des Auges gemäss Fig.1 mit einem innerhalb des ersten parabolischen Einschnitts angeordneten zweiten parabolischen Einschnitt sowie aufgeklapptem zweiten Skleralappen;
- **Fig.4**: das entlang der Linie IV-IV im Schnitt dargestellte Teilstück des Auges gemäss Fig.3 mit den beiden aufgeklappten Skleralappen und freigelegtem Schlemmschen Kanal;
- **Fig.5**: eine in das freigelegte Teilstück des Schlemmschen Kanals eingeführte Vorrichtung zum Injizieren eines das Lumen des Schlemmschen Kanals dehnenden Mediums;
- **Fig.6**: das Teilstück des Auges gemäss Fig.4 mit einem im Bereich der beiden aufgeklappten Skleralappen mit geringer Kraft gegen die Schwalbesche Linie wirkenden Tupfer;
- **Fig.7**: das Teilstück des Auges gemäss Fig.6 bei welchem die Descemet-Membran von der Kornea gelöst und der zweite Skleralappen abgetrennt ist;
- **Fig.8**: das Teilstück des Auges gemäss Fig.4 mit in das Lumen des Schlemmschen Kanals implantierten, beispielsweise ringförmig oder kugelförmig ausgebildeten Stützelementen;
- **Fig.9**: das Teilstück des Auges gemäss Fig.4 mit in das Lumen des Schlemmschen Kanals implantierten, beispielsweise in Form eines Netzgeflecht oder einer Schraubenfeder ausgebildeten Stützelementen;
- **Fig.10**: einzelne Ausführungsbeispiele der zu implantierenden Stützelemente;
- **Fig.10A**: das in räumlicher Ansicht sowie in grösserem Massstab dargestellte und ringförmig ausgebildete Stützelement;
- **Fig.10B**: das in räumlicher Ansicht sowie in grösserem Massstab dargestellte und kugelförmig ausgebildete Stützelement;
- **Fig.10C**: ein in Ansicht sowie in grösserem Massstab dargestelltes und röhrchenförmig ausgebildetes Stützelement;
- **Fig.10D**: ein in Ansicht sowie in grösserem Massstab dargestelltes und röhrchenförmig ausgebildetes Stützelement für das freigelegte Teilstück des Schlemmschen Kanals;
- **Fig.10E**: das gemäss der Linie E-E im Schnitt dargestellte Stützelement gemäss Fig.10D;
- **Fig.10F**: ein in Ansicht sowie in grösserem Massstab dargestelltes Stützelement in Form eines Netzgeflechts;
- **Fig.10G**: ein in Ansicht sowie in grösserem Massstab dargestelltes Stützelement in Form einer Schraubenfeder;
- **Fig.11**: das Teilstück des Auges gemäss Fig.8 mit implantierten Stützelementen gemäss Fig.10A sowie mit dem im freigelegten Teilstück des Schlemmschen Kanals implantierten Stützelement gemäss Fig.10D;
- **Fig.12**: das gemäss der Linie XII-XII in Fig.11 im Schnitt dargestellte Teilstück des Auges mit dem gemäss Fig.10E im Schnitt dargestellten Stützelement und heruntergeklapptem ersten Skleralappen;
- **Fig.13**: das Teilstück des Auges gemäss Fig.8 mit den implantierten Stützelementen gemäss Fig.10A sowie dem im freigelegten Teilstück des Schlemmschen Kanals implantierten Stützelement gemäss Fig.10F; und
- **Fig.14**: das Teilstück des Auges gemäss Fig.8 mit den implantierten Stützelementen gemäss Fig.lOA und Fig.10B sowie dem im freigelegten Teilstück des Schlemmschen Kanals implantierten Stützelement gemäss Fig.10G.

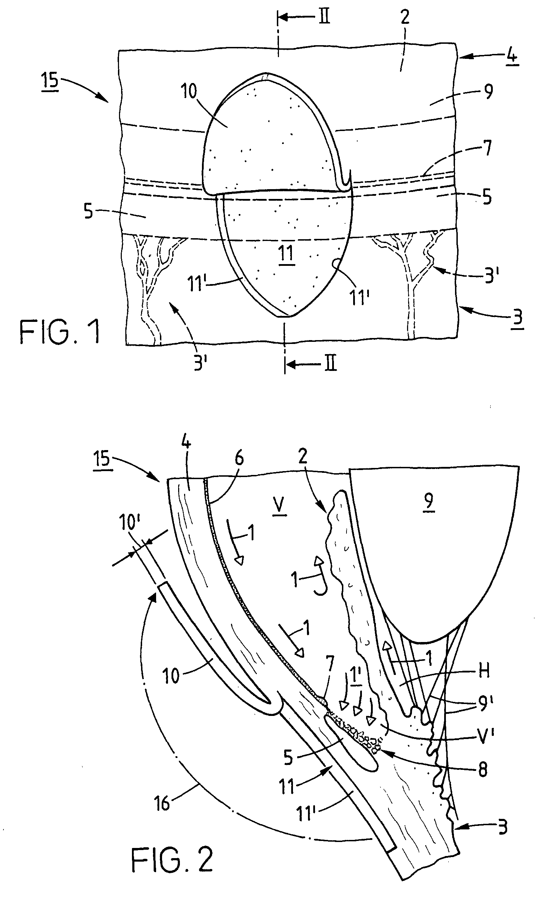

Fig.1 zeigt ein in grösserem Massstab sowie in schematischer Ansicht dargestelltes Teilstück eines in der Gesamtheit mit 15 bezeichneten Auges und man erkennt jeweils ein Teilstück der Regenbogenhaut 2 (Iris), der Hornhaut 4 (Kornea), der Lederhaut 3 (Sklera), ein Teilstück des zirkulären Schlemmschen Kanals 5 (sinus venosus sclerae) und das aus einer Vielzahl von Kanälchen gebildete Kanalsystem 3' für das abzuleitende Kammerwasser. Weiterhin erkennt man in Fig.1 einen ersten etwa parabolisch ausgebildeten Einschnitt (Inzision) in der Sklera 3, einen analog dem Einschnitt ausgebildeten und in Richtung der Kornea 4 aufgeschwenkten ersten Skleralappen 10 sowie eine analog demselben ausgebildete erste Ausnehmung 11 mit umlaufender Seitenwand 11'. Der erste Skleralappen 10 ist durch nicht dargestellte Mittel in der aufgeschwenkten Position gehalten.

In Fig.2 ist das Teilstück des Auges 15 gemäss der in Fig.1 eingezeichneten Linie II-II im Schnitt sowie in grösserem Massstab dargestellt und man erkennt ein Teilstück der Sklera 3, ein Teilstück der Kornea 4 mit der Descemet-Membran 6 (Descemet's membrane) und der Schwalbeschen Linie 7 (Schwalbe's line), ein Teilstück der Regenbogenhaut 2 sowie ein Teilstück der Linse 9, welches mittels der Zonularfasern 9' mit der Sklera 3 verbunden ist. Weiterhin erkennt man den gemäss Pfeilrichtung 16 aufgeschwenkten ersten Skleralappen 10 mit der analog ausgebildeten ersten Ausnehmung 11 sowie den Schlemm'schen Kanal 5 mit dem vorgelagerten Trabekulargewebe 8 (trabecular meshwork).

In Fig.2 ist weiterhin mit mehreren Pfeilen 1 etwa die Zirkulation des Kammerwassers (humor aquosus) und mit den Pfeilen 1' der natürliche Abfluss über das Trabekulargewebe 8 dargestellt. Bei einem gesunden Auge zirkuliert das sich ständig erneuernde Kammerwasser gemäss Pfeilrichtung 1 von der Hinterkammer H zur Vorderkammer V und wird im Kammerwinkel V' (angulus irido-cornealis) gemäss der Pfeile 1' im wesentlichen durch das Trabekulargewebe 8 in den Schlemmschen Kanal 5 und von dort über das natürliche Kanalsystem 3' (Fig.1) in ein nicht dargestelltes Venensystem abgeleitet. Bei einem infolge Verstopfung oder dergleichen nur teilweise funktionsfähigen beziehungsweise funktionsunfähigen Trabekulargewebe 8 kann der natürliche Abfluss des Kammerwassers derart begrenzt werden, dass der Druck im Inneren des Auges 15 ansteigt und infolge dessen die Durchblutung und somit die Funktion der Sehnerven (nicht dargestellt) eingeschränkt wird. Diese Krankheit ist allgemein unter dem Begriff "Glaukom" bekannt, welche zur Erblindung des betroffenen Auges führen kann.

Für den mikrochirurgischen Eingriff wird die nicht dargestellte Bindehaut des Auges mit geeigneten Mitteln zurückgezogen und dadurch ein ausreichendes Teilstück der Sklera 3 für den ersten etwa parabolförmigen Einschnitt (Inzision) freigelegt. Nach dem ersten Einschnitt wird der dem Einschnitt entsprechend ausgebildete erste Skleralappen 10 in Richtung der Kornea 4 geklappt und infolge dessen die erste Ausnehmung 11 mit der umlaufenden Seitenwand 11' freigelegt. Die Tiefe des ersten beispielsweise 3mm x 3mm grossen Einschnitts ist vorzugsweise so gewählt, dass die Dicke 10' des in Fig.2 im Profilquerschnitt dargestellten ersten Skleralappens 10 etwa 1/3 der in diesem Bereich natürlichen Dicke der Sklera 3 entspricht. Der Schlemmsche Kanal 5 ist in dieser ersten Phase (Fig.2) noch von der Sklera 3 verdeckt.

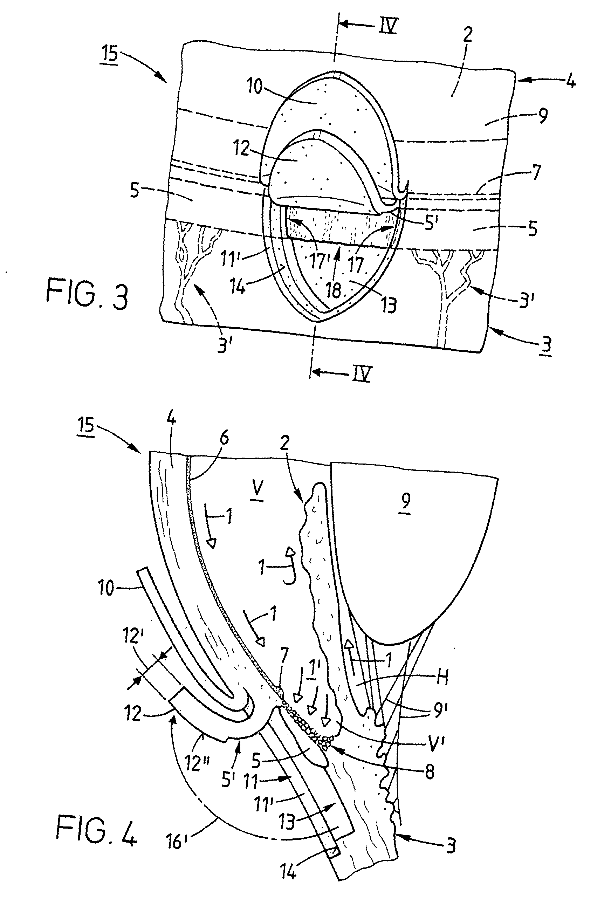

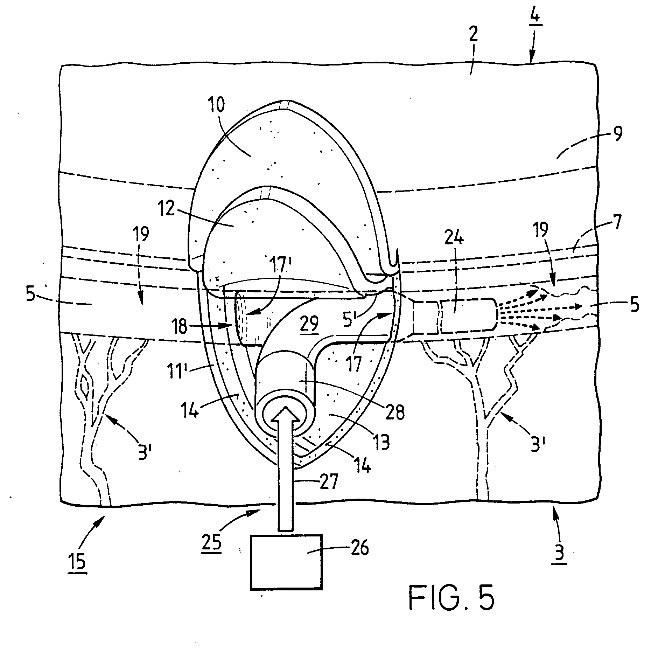

Fig.3 zeigt das Teilstück des Auges 15 gemäss Fig.1 mit einem innerhalb des ersten parabolischen Einschnitts angeordneten zweiten parabolischen Einschnitt. Nach Durchführung des zweiten Einschnitts wird ein entsprechend parabolförmig ausgebildeter zweiter Skleralappen 12 in Richtung der Kornea 4 aufgeklappt und dabei eine analog des zweiten Skleralappens 12 ausgebildete zweite Ausnehmung 13 freigelegt. Die Tiefe des zweiten Einschnitts in der Sklera 3 ist beispielsweise so gewählt, dass bei aufgeschwenktem zweiten Skleralappen 12 der Schlemmsche Kanal 5 im Bereich eines freigelegten und in der Gesamtheit mit 18 bezeichneten Teilstücks zugänglich ist. In dieser Phase sind die beiden innerhalb des freigelegten Teilstücks 18 gegenüberliegend im Abstand zueinander angeordneten Eingänge 17 und 17' des Schlemmschen Kanals 5 zum Einführen einer entsprechend ausgebildeten Sonde (Fig.5) zugänglich. In Fig.3 ist weiterhin die in Form einer sich über die gesamte Breite des zweiten Skleralappens 12 erstreckende etwa rillenförmige Vertiefung 5' des Schlemmschen Kanals 5 dargestellt.

In Fig.4 ist das Teilstück des Auges 15 gemäss der in Fig.3 eingezeichneten Linie IV-IV mit den beiden gemäss Pfeilrichtung 16 (Fig.3) und Pfeilrichtung 16' (Fig.4) aufgeschwenkten Skleralappen 10 und 12 im Schnitt dargestellt. Die beiden Skleralappen 10,12 sind für den weiteren Eingriff mit nicht dargestellten Mitteln in dieser Position gehalten. Die Schnitttiefe sowie die davon abhängige Dicke 12' des in Fig.4 im Profilquerschnitt dargestellten zweiten Skleralappens 12 ist beispielsweise so gewählt, dass die beiden Eingänge 17,17' (Fig.3) über das freigelegte Teilstück 18 gut zugänglich sind. Dies wird im wesentlichen dadurch erreicht, dass infolge der gewählten Schnitttiefe, wie vorstehend erwähnt an der Innenseite 12" des zweiten Skleralappens 12 noch das Teilstück des Schlemmschen Kanals 5 in Form der rillenförmigen Vertiefung 5' vorhanden ist. Weiterhin erkennt man in Fig.3 und Fig.4 die beiden Ausnehmungen 11 und 13 in der Sklera 3 mit der Seitenwand 11' und der Auflagefläche 14.

In einer nächsten Phase gemäss Fig.5 wird vorzugsweise mittels einer entsprechend ausgebildeten Injektionsvorrichtung 25 in die beiden seitlichen Eingänge 17 und 17' des freigelegten Teilstücks 18 des Schlemmschen Kanals 5 ein geeignetes Medium, vorzugsweise eine hochviscose Natrium-Hyaluronat-Lösung (high viscosity sodium hyaluronate) injiziert. Hierdurch wird das Lumen 19 des Schlemmschen Kanals 5 mindestens über die axiale Länge einer eingeführten Sonde 24 aufgeweitet. In Fig.5 ist das Teilstück des Auges 15 gemäss Fig.3 in grösserem Massstab dargestellt und man erkennt die teilweise dargestellte Sklera 3 mit den beiden in Richtung der Kornea 4 aufgeschwenkten beziehungsweise hochgeklappten Skleralappen 10 und 12, die zweite Ausnehmung 13 sowie die seitliche Auflagefläche 14 an der Sklera 3 mit dem Kanalsystem 3'.

Zum Injizieren der hochviscosen Natrium-Hyaluronat-Lösung ist in Fig.5 die Injektionsvorrichtung 25 schematisch dargestellt, welche bei dem dargestellten Ausführungsbeispiel mit der an einem bogenförmigen Anschlussteil 29 angeordneten Sonde 24 in den Eingang 17 des Schlemmschen Kanals 5 eingeführt ist. Nach dem Aufweiten des Lumens 19 auf der einen Seite wird die Injektionsvorrichtung 25 mit der Sonde 24 herausgezogen und in nicht näher dargestellter Weise entsprechend gedreht in den anderen, gegenüberliegenden Eingang 17' des Schlemmschen Kanals 5 zum Injizieren des Mediums beziehungsweise zum Aufweiten des Lumens 19 eingeführt.

Die in Fig.5 als Ausführungsbeispiel dargestellte Injektionsvorrichtung 25 steht über eine daran angeschlossene Zuführleitung 28 mit einer schematisch dargestellten Druckquelle 26 in Form einer Einkammer-Spritze oder dergleichen in Verbindung. Mittels der beispielsweise manuell oder elektrisch betätigbaren Druckquelle 26 wird das zu injizierende Medium gemäss Pfeilrichtung 27 durch die Elemente 28,29 und 24 in das Lumen 19 des Schlemmschen Kanals 5 gepresst und dieser entsprechend gedehnt. Nach dem Aufweiten des Schlemmschen Kanals 5 wird die Injektionsvorrichtung 25 in nicht dargestellter Weise wieder entfernt. Die Injektionsvorrichtung 25 ist nicht Gegenstand vorliegender Erfindung und wird deshalb nicht näher beschrieben.

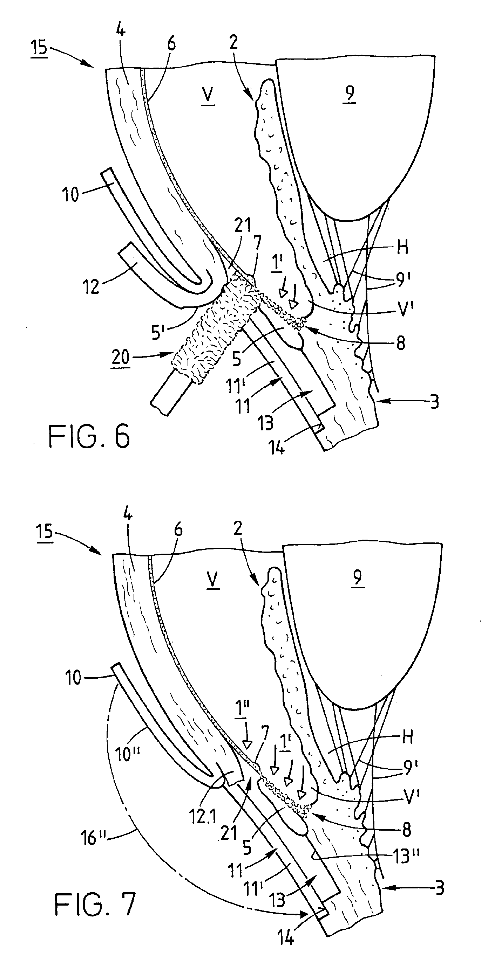

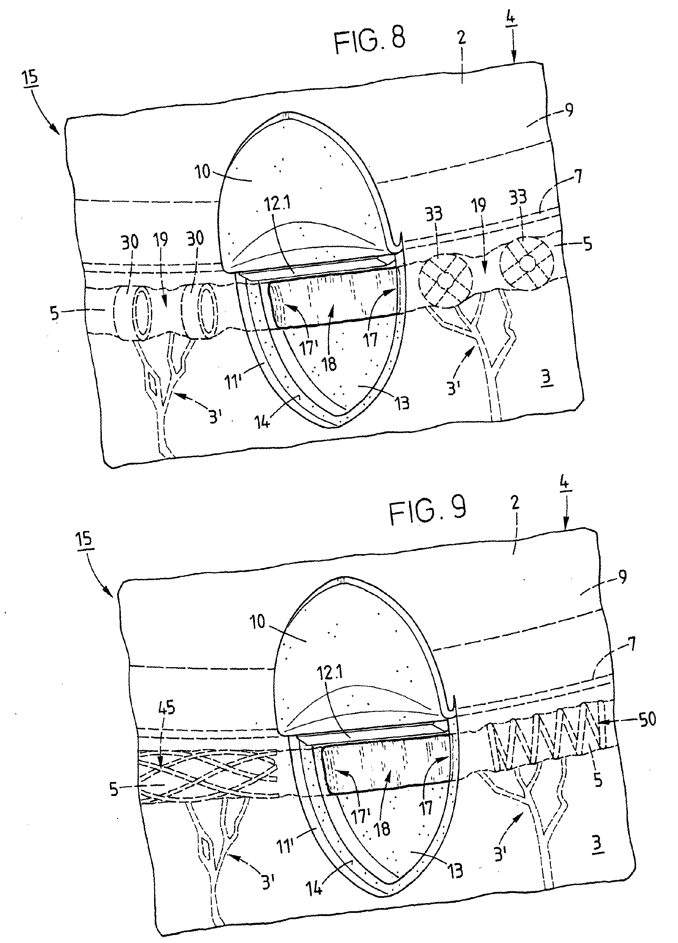

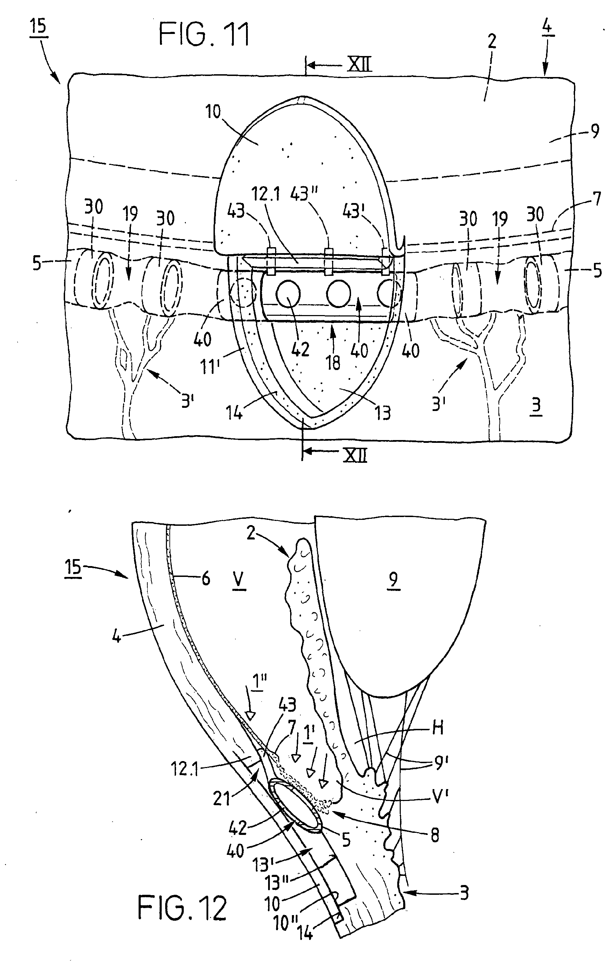

Wie in Fig.6 und Fig.7 schematisch dargestellt, kann beispielweise vor dem Implantieren entsprechend ausgebildeter Stützelemente mittels eines Tupfers 20 oder dergleichen durch eine geringe Kraft im Bereich der Schwalbeschen Linie 7 der zweite Skleralappen 12 von der zugewandten Innenseite der Descemet-Membran 6 gelöst werden. Hierdurch entsteht zwischen dem zweiten Skleralappen 12 und der Descemet-Membran 6 eine in Fig.7 schematisch dargestellte Öffnung 21. Die etwa spaltförmige Öffnung 21 erstreckt sich in nicht dargestellter Weise über die gesamte Breite des freigelegten Teilstücks 18 beziehungsweise der zweiten Ausnehmung 13 (Fig.8). Mit der in Fig.7 dargestellten spaltförmigen Öffnung 21 (window) wird eine zusätzliche Verbindung zwischen der Vorderkammer V des Auges 15 und der zweiten Ausnehmung 13 hergestellt. Das Kammerwasser wird somit ergänzend zu dem natürlichen Abfluss über das Trabekulargewebe 8 gemäss Pfeilrichtung 1' zusätzlich noch durch die weitgehend transparente und teilweise kammerwasserdurchlässige Descemet-Membran 6 gemäss Pfeilrichtung 1" (Fig.7) und der spaltförmigen Öffnung 21 in die Ausnehmung 13 abgeleitet. Die etwa analog dem zweiten Skleralappen 12 ausgebildete flächige Ausnehmung 13 bildet bei heruntergeklapptem Skleralappen 10 im wesentlichen ein Sammelbekken (Fig.12) für das Kammerwasser. Das Kammerwasser wird von der als Sammelbecken (Reservoir) ausgebildeten Ausnehmung 13 durch die beiden seitlichen Eingänge 17,17' in den Schlemmschen Kanal 5 und von dort über das Kanalsystem 3' (Fig.1 und Fig.3) abgeleitet.

Der zweite Skleralappen 12 wird vorzugsweise, wie in Fig.7 schematisch dargestellt, bis auf ein restliches Teilstück 12.1 mittels eines geeigneten chirurgischen Schneidinstruments abgetrennt. Es besteht jedoch auch die Möglichkeit, dass zuerst der zweite Skleralappen 12 abgetrennt und anschliessend mittels des gegen die Schwalbesche Linie 7 gedrückten Tupfers 20 das verbleibende Teilstück 12.1 von der zugewandten Innenseite der Descemet-Membran 6 zur Bildung der spaltförmigen Öffnung 21 gelöst wird.

Fig.8 und Fig.9 zeigen jeweils das Teilstück des Auges 15 mit abgetrenntem Skleralappen 12 und man erkennt das freigelegte Teilstück 18 des Schlemmschen Kanals 5 sowie die beiden gegenüberliegend zueinander angeordneten Eingänge 17 und 17'. In das Lumen 19 des Schlemmschen Kanals 5 sind zu beiden Seiten des freigelegten Teilstücks 18 jeweils zwei im Abstand zueinander angeordnete Stützelemente implantiert. Bei dem einen Ausführungsbeispiel gemäss Fig.8 sind auf der einen Seite zwei etwa ringförmig ausgebildete Stützelemente 30 und auf der gegenüberliegenden anderen Seite zwei kugelförmige Stützelemente 33 implantiert. Bei dem anderen Ausführungsbeispiel gemäss Fig.9 ist auf der einen Seite ein als Netzgeflecht ausgebildete Stützelement 45 und auf der gegenüberliegenden anderen Seite ein als Schraubenfeder ausgebildetes Stützelement 50 implantiert.

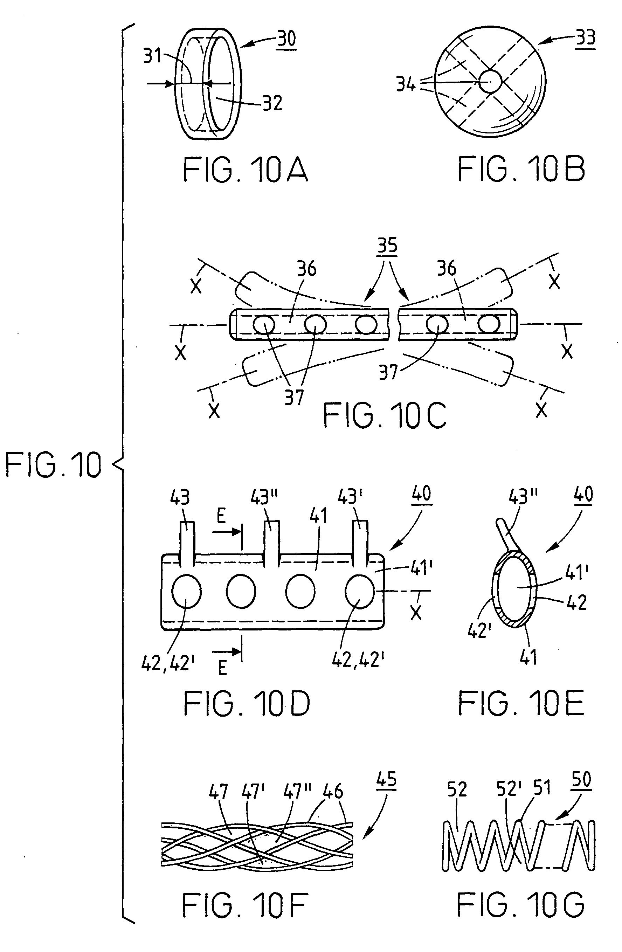

Nachstehend werden mehrere Ausführungsbeispiele der Stützelemente 30,33,35,40,45 und 50 in Verbindung mit Fig.10 im einzelnen beschrieben, wobei an dieser Stelle darauf hingewiesen wird, dass die einzelnen Stützelemente miteinander in beliebiger Kombination in das Lumen 19 des Schlemmschen Kanals 5 implantierbar sind.

In Fig.10A ist das erste Stützelement 30 in räumlicher Ansicht sowie in grösserem Massstab dargestellt. Das von einer Bohrung 32 durchdrungende Stützelement 30 hat vorzugsweise eine etwa kreisring- oder ellipsenringförmige äussere Formgebung. Das beispielsweise kreisringförmig ausgebildete Stützelement 30 ist vorzugsweise aus einem sich selbsttätig an das Lumen 19 des Schlemmschen Kanals 5 anpassenden Material hergestellt. Die Breite 31 des kreisring- oder ellipsenringförmigen Stützelements 30 ist beispielsweise so gewählt, dass dasselbe lagestabil im Lumen 19 implantierbar ist und ein in axialer Richtung des Lumens 19 orientiertes Umkippen des Stützelements 30 ausgeschlossen ist.

In Fig.10B ist das zweite kugelförmig ausgebildete Stützelement 33 in räumlicher Ansicht sowie in grösserem Massstab dargestellt. Das kugelförmige Stützelement 33 wird von mindestens einer, vorzugsweise aber von mehreren in Umfangsrichtung verteilt zueinander angeordneten Bohrungen 34 durchdrungen.

In Fig.10C ist das dritte Stützelement 35 in Ansicht dargestellt, welches beispielsweise aus einem flexiblen Röhrchen hergestellt ist und im Profilquerschnitt (nicht dargestellt) entweder eine kreisring- oder ellipsenförmige äussere Formgebung aufweist. Das Stützelement 35 wird in axialer Richtung von einer Bohrung 36 durchdrungen. Die Bohrung 36 steht mit einer Anzahl in axialer Richtung im Abstand zueinander angeordneten Einrittsöffnungen 37 in Verbindung. Das Stützelement 35 ist in Bezug auf die theoretische Längsachse X hinsichtlich seiner Lage und Orientierung wie in Fig.10C schematisch dargestellt frei beweglich ausgebildet. Das Stützelement 35 kann sich beim Einführen in den Schlemmschen Kanal 5 infolge der Flexibilität selbsttätig an die innere Formgebung des Lumens 19 anpassen. Die Flexibilität des röhrchenförmigen Stützelements 35 ist jedoch so begrenzt, dass ein selbsttätiges Abknicken ausgeschlossen ist.

Fig.10D zeigt das in Ansicht sowie in grösserem Massstab dargestellte vierte Stützelement 40, welches ebenfalls als längliches, flexibles Röhrchen 41 ausgebildet ist. Das Stützelement 40 wird von einer in axialer Richtung orientierten Bohrung 41' durchdrungen, wobei die Bohrung 41' mit einer Anzahl in axialer Richtung im Abstand zueinander angeordneten Öffnungen 42,42' in Verbindung steht. An dem Stützelement 40 sind weiterhin mehrere in axialer Richtung im Abstand zueinander angeordnete Auflageteile 43,43' und 43" angeordnet, vorzugsweise angeformt. Die mit der in axialer Richtung orientieren Bohrung 41' in Verbindung stehenden Öffnungen 42,42' sind vorzugsweise diametral zueinander an der längeren Seite (nicht bezeichnet) des im Profilquerschnitt ellipsenförmigen Stützelements 40 angeordnet. Das Stützelement 40 ist insbesondere zum Implantieren in das im Bereich der zweiten Ausnehmung 13 der Sklera 3 freigelegte Teilstück 18 des Schlemmschen Kanals 5 ausgebildet. Das entlang der in Fig.10D eingezeichneten Linie E-E im Schnitt gemäss Fig.10E dargestellte Stützelement 40 hat eine etwa dem Profilquerschnitt des Schlemmschen Kanals 5 entsprechend ausgebildete elliptische äussere Formgebung. Die Funktion des vierten Stützelements 40 wird nachstehend in Verbindung mit Fig.11 und Fig.12 noch näher beschrieben.

In Fig.10F ist als weitere Variante das als Netzgeflecht ausgebildete Stützelement 45 in Ansicht dargestellt. Bei dem aus einer Vielzahl von Fäden 46 (Filamente) schraubenlinienförmig gewundenen Netzgeflecht sind zwischen den Fäden 46 entsprechende Abstände 47,47' und 47" vorgesehen, durch welche das Kammerwasser abgeleitet werden kann. Das Stützelement 45 ist vorzugsweise analog dem in Fig.10C dargestellten Stützelement 35 in Bezug auf seine äussere Formgebung an das Lumen 19 des Schlemmschen Kanals 5 selbstanpassend verformbar.

Fig.10G zeigt das in Ansicht sowie in grösserem Massstab dargestellte Stützelement 50, welches beispielsweise aus einem einzigen, schraubenlinienförmig gewundenen Faden 51 (Filament) hergestellt ist. Bei dem Stützelement 50 wird das Kammerwasser durch die zwischen den einzelnen Windungen vorgesehenen Abstände 52 und 52' abgeleitet. Das Stützelement 50 ist vorzugsweise analog dem in Fig.10C dargestellten Stützelement 35 in Bezug auf seine äussere Formgebung an das Lumen 19 des Schlemmschen Kanals 5 selbstanpassend verformbar.

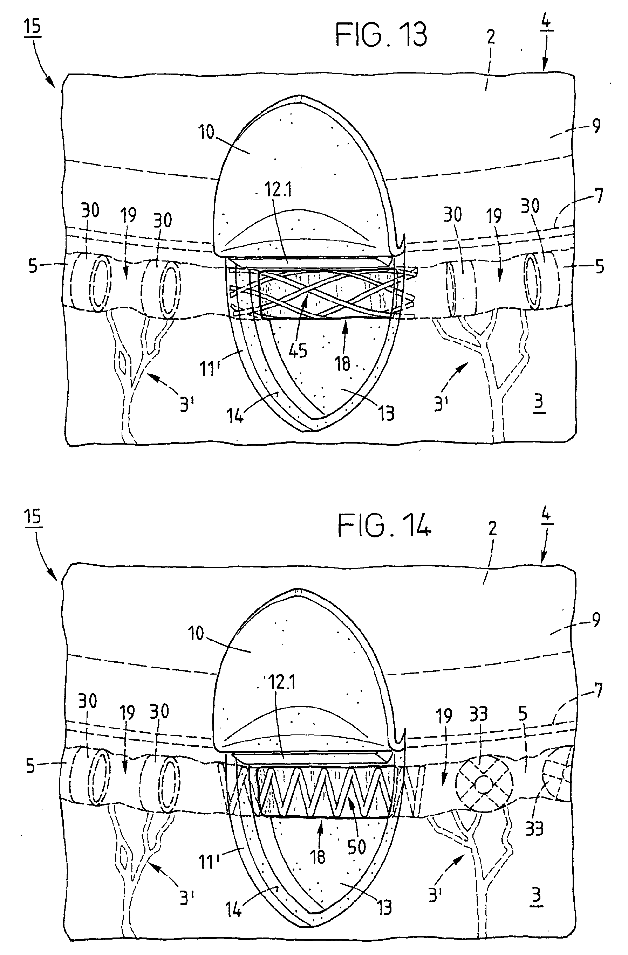

Die vorstehend in Verbindung mit den Figuren 10C,10F und 10G beschriebenen länglichen Stützelemente 35,40,45 oder 50 können sowohl in das im Bereich der zweiten Ausnehmung 13 der Sklera 3 freigelegte Teilstück 18 des Schlemmschen Kanals 5 oder in das Lumen 19 desselben implantiert werden. Eine Implantation der länglichen Stützelemente 35,45 und 50 in das freigelegte Teilstück 18 des Schlemmschen Kanals 5 ist ebenfalls möglich (Fig.13 und Fig.14).

Fig.11 zeigt das Teilstück des Auges 15 mit den zu beiden Seiten der zweiten Ausnehmung 13 in das Lumen 19 des Schlemmschen Kanals 5 im Abstand zueinander angeordneten und ringförmig ausgebildeten Stützelementen 30. Weiterhin erkennt man das im Bereich der zweiten Ausnehmung 13 der Sklera 3 in den freigelegten Schlemmschen Kanal 5 eingeführte Stützelement 40. Das Stützelement 40 ist derart in dem freigelegten Teilstück 18 des Schlemmschen Kanals 5 angeordnet, dass das über die gesamte Breite der zweiten Ausnehmung 13 von dem zweiten Skleralappen 12 gelöste Teilstück 12.1 auf den Auflageteilten 43,43',43" des Stützelements 40 liegt.

Nach dem Abtrennen des zweiten Skleralappens 12 und dem Implantieren der einzelnen Stützelemente wird der erste Skleralappen 10 herunter geklappt und wie in Fig.12 schematisch dargestellt auf die parabolförmig umlaufende Auflagefläche 14 gelegt. Anschliessend wird der erste Skleralappen 10 in an sich bekannter, nicht näher dargestellter Weise teilweise mit der Sklera 3 vernäht. Der infolge des abgetrennten zweiten Skleralappens 12 hinter dem ersten Skleralappen 10 entstandene subsklerale Raum 13' in Form der flächigen Ausnehmung 13 wird vorzugsweise vor dem vollständigen Vernähen noch mit hochviscosem Medium (high viscosity sodium hyaluronate) gefüllt. Hierdurch wird verhindert, dass ein verbindender Kontakt der Innenseites 10" des heruntergeklappten erste Skleralappen 10 mit der Innenfläche 13" der Ausnehmung 13 erfolgt.

In Fig.12 ist das Teilstück des Auges 15 mit dem implantierten Stützelement 40 gemäss der in Fig.11 eingezeichneten Linie XII-XII im Schnitt sowie in grösserem Massstab sowie mit heruntergeklapptem Skleralappen 10 dargestellt. Weiterhin erkennt man in Fig.12 das von der Descemet-Membran 6 gelöste und auf den Auflageteilten 43,43',43" des Stützelements 40 liegende Teilstück 12.1 des zweiten Skleralappens 12. Mittels der Auflageteile 43,43',43" wird ein Verschliessen der spaltförmigen Öffnung 21 über die gesamte Breite des freigelegten Teilstücks 18 des Schlemmschen Kanals 5 verhindert.

Wie in Fig.12 dargestellt, wird mit der spaltförmigen Öffnung 21 eine zusätzliche Verbindung zwischen dem Kammerwinkel V' der Vorderkammer V und der zweiten Ausnehmung 13 hergestellt. Das Kammerwasser kann somit zusätzlich zu dem natürlichen Abfluss gemäss Pfeilrichtung 1' über das Trabekulargewebe 8 auch noch gemäss Pfeilrichtung 1" durch die weitgehend transparente und teildurchlässige Descemet-Membran 6 und durch die spaltförmige Öffnung 21 (window) in die Ausnehmung 13 abgeleitet werden. Die etwa analog dem zweiten Skleralappen 12 ausgebildete flächige Ausnehmung 13 bildet bei heruntergeklapptem Skleralappen 10 einen subskleralen Raum 13' oder ein Sammelbecken (Reservoir) für das Kammerwasser. Das Kammerwasser wird von dem subskleralen Raum 13' durch die beiden damit in Verbindung stehenden seitlichen Eingänge 17,17' in das Lumen 19 des Schlemmschen Kanals 5 und von dort in das Kanalsystem 3' abgeleitet.

Fig.13 zeigt das Teilstück des Auges 15 mit in das Lumen 19 des Schlemmschen Kanals 5 im Abstand zueinander implantierten, ringförmig ausgebildeten Stützelementen 30 (Fig.10A). Anstelle oder in Kombination können in das Lumen 19 jedoch auch mehrere im Abstand zueinander angeordnete und kugelförmig ausgebildete Stützelemente 33 (Fig.10B) implantiert werden. Weiterhin besteht die Möglichkeit, in dem freigelegten Teilstück 18 des Schlemmschen Kanals 5 das als Netzgeflecht ausgebildete Stützelement 45 (Fig.10F) in das freigelegte Teilstück 18 des Schlemmschen Kanals 5 zu implantieren.

Fig.14 zeigt das Teilstück des Auges 15 mit Schlemmschen Kanal 5, bei welchem auf der einen Seite in das Lumen 19 beispielsweise zwei ringförmige und im Abstand zueinander angeordnete Stützelemente 30 gemäss Fig.10A implantiert sind. Auf der anderen gegenüberliegenden Seite sind in das Lumen 19 zwei im Abstand zu einander angeordnete und kugelförmig ausgebildete Stützelemente 33 gemäss Fig.10B implantiert. Bei diesem Ausführungsbeispiel ist in dem freigelegten Teilstück 18 des Schlemmschen Kanals 5 das als Schraubenfeder ausgebildete Stützelement 50 gemäss Fig.10G implantiert.

Bei einer nicht dargestellten Variante besteht jedoch auch die Möglichkeit, dass auf der einen Seite des freigelegten Teilstücks 18 zwei oder mehrere ringförmige Stützelemente 30 oder 33 und auf der gegenüberliegenden Seite ein oder mehrere längliche Stützelemente 35,45,50 in das Lumen 19 des Schlemmschen Kanals 5 implantiert werden. Bei einer weiteren Variante besteht zudem die Möglichkeit in das freigelegte Teilstück 18 des Schlemmschen Kanals 5 zusätzlich noch das längliche Stützelement 40 gemäss Fig.10D und Fig.10E zu implantieren.

An dieser Stelle wird darauf hingewiesen, dass mindestens die jeweils in das Lumen 19 des Schlemmschen Kanals 5 implantierbaren Stützelemente 30,33,35,45 oder 50 aus abbaubarem, insbesondere aus einem von dem Gewebe des Schlemmschen Kanals 5 und/oder von dem Kammerwasser biolytisch abbaubarem Material hergestellt sind. Als Material wird vorzugsweise eine Werkstoffgruppierung gewählt, welche etwa innerhalb von 2 bis 12 Monaten nach der Implantation biolytisch abbaubar ist.

Zur Herstellung des einzelnen Stützelements 30,33,35,45 oder 50 kann ein Werkstoff wie beispielsweise ein vernetztes Natriumhyaluronat (cross linked sodium hyaluronate) verwendet werden.

Es besteht jedoch auch die Möglichkeit, dass insbesondere das längliche in das freigelegte Teilstück 18 des Schlemmschen Kanals 5 zu implantierende Stützelement 35,40,45 oder 50 aus biokompatiblem Material, beispielsweise aus Kunststoff, nichtrostendem Stahl oder Edelstahl, wie Silber, Gold oder Platin hergestellt ist.

Die vorstehend in Verbindung mit den einzelnen Figuren beschriebenen Stützelemente und deren abbaubaren Materialien sind nicht auf die dargestellten und erwähnten Ausführungsbeispiele beschränkt. Weitere zweckmässige Ausgestaltungen und abbaubare Materialen ohne dabei den Grundgedanken der Erfindung zu verlassen sind ebenfalls möglich.

## Patentansprüche

1. Verfahren zum Verbessern des Kammerwasserabflusses in einem Auge (15), bei welchem das abgesonderte Kammerwasser im Bereich des Kammerwinkels (V') der Vorderkammer (V) über das Trabekulargewebe (8) in den Schlemmschen Kanal (5) und anschliessend über das natürliche Kanalsystem abgeleitet wird, **gekennzeichnet durch** einen ersten lamellaren Einschnitt an der Oberfläche der Sklera (3) mit in Richtung der Kornea (4) aufgeschwenktem ersten Skleralappen (10) sowie analog ausgebildeter erster Ausnehmung (11), einen innerhalb der ersten Ausnehmung (11) vorgesehenen zweiten Einschnitt mit aufgeschwenktem zweiten Skleralappen (12) und analog ausgebildeter zweiter Ausnehmung (13) sowie ein in diesem Bereich freigelegtes Teilstück (18) des Schlemmschen Kanals (5), wobei zu beiden Seiten der zweiten Ausnehmung (13) in das Lumen (19) sowie wahlweise in das freigelegte Teilstück (18) jeweils mindestens ein von dem Gewebe des Schlemmschen Kanals 5 und/oder vom Kammerwasser abbaubares Stützelement implantiert und nach dem Abtrennen des zweiten Skleralappens (12) der erste Skleralappen (10) auf eine analog dem zweiten Einschnitt ausgebildete Auflagefläche (14) gelegt und der dadurch gebildete subsklerale Raum (13') vor dem vollständigen Verschliessen mit viskosem Medium gefüllt wird.

2. Verfahren nach Anspruch 1, **dadurch gekennzeichnet,** dass das Lumen (19) zu beiden Seiten des freigelegten Teilstücks (18) von mindestens zwei in axialer Richtung des Schlemmschen Kanals (5) im Abstand zueinander angeordneten und etwa ringförmig ausgebildeten Stützelementen (30) aus biolytisch abbaubarem Material in gedehnter Stellung gehalten wird.

3. Verfahren nach Anspruch 1, **dadurch gekennzeichnet,** dass das Lumen (19) zu beiden Seiten des freigelegten Teilstücks (18) von mindestens zwei in axialer Richtung des Schlemmschen Kanals (5) im Abstand zueinander angeordneten und etwa kugelförmig ausgebildeten Stützelementen (33) aus biolytisch abbaubarem Material in gedehnter Stellung gehalten wird.

4. Verfahren nach Anspruch 1, **dadurch gekennzeichnet,** dass das Lumen (19) des Schlemmschen Kanals (5) zu beiden Seiten des freigelegten Teilstücks (18) jeweils von mindestens einem länglichen, röhrchenförmig ausgebildeten Stützelement (35) aus biolytisch abbaubarem Material in gedehnter Stellung gehalten wird.

5. Verfahren nach Anspruch 1, **dadurch gekennzeichnet,** dass das Lumen (19) des Schlemmschen Kanals (5) zu beiden Seiten des freigelegten Teilstücks (18) jeweils von mindestens einem länglichen, als Netzgeflecht ausgebildeten Stützelement (45) aus biolytisch abbaubarem Material in gedehnter Stellung gehalten wird.

6. Verfahren nach Anspruch 1, **dadurch gekennzeichnet,** dass das Lumen (19) des Schlemmschen Kanals (5) zu beiden Seiten des freigelegten Teilstücks (18) jeweils von mindestens einem länglichen, als Schraubenfeder ausgebildeten Stützelement (50) aus biolytisch abbaubarem Material in gedehnter Stellung gehalten wird.

7. Verfahren nach einem der Ansprüche 1 bis 6, **dadurch gekennzeichnet,** dass das Lumen (19) zu beiden Seiten des freigelegten Teilstücks (18) jeweils mittels ring- und/oder kugelförmiger sowie im Abstand zueinander angeordneter Stützelemente (30;33) und das freigelegte Teilstück (18) des Schlemmschen Kanals (5) durch ein längliches Stützelement (35;45;50) jeweils aus biolytisch abbaubarem Material in gedehnter Stellung gehalten wird.

8. Verfahren nach Anspruch 1, **dadurch gekennzeichnet,** dass zur Bildung einer spaltförmigen Öffnung (21) der zweite Skleralappen (12) oder ein Teilstück (12.1) desselben im Bereich der Schwalbeschen Linie (7) von der teilweise kammerwasserdurchlässigen Descemet-Membran (6) gelöst wird, wobei das abgelöste Teilstück (12.1) mittels mehrerer Auflageteile (43, 43',43") eines in dem freigelegten Teilstück (18) des Schlemmschen Kanals (5) angeordneten Stützelements (40) in Offenstellung gehalten wird.

9. Vorrichtung zum Verbessern und zur Aufrechterhaltung des Kammerwasserabflusses in einem Auge (15), bei welchem im Lumen (19) des Schlemmschen Kanals (5) mindestens ein Stützelement angeordnet ist, **gekennzeichnet durch** mindestens ein ringförmig, kugelförmig oder röhrchenförmig ausgebildetes und im Lumen (19) des Schlemmschen Kanals (5) angeordnetes Stützelement (30;33;35;45;50), welches aus abbaubarem Material, insbesondere aus einem von dem Gewebe des Schlemmschen Kanals (5) und/oder von dem Kammerwasser abbaubarem Material hergestellt ist.

10. Vorrichtung nach Anspruch 9, **dadurch gekennzeichnet,** dass das mit einer Durchgangsbohrung (31) versehene Stützelement (30) im Profilquerschnitt kreisring- oder ellipsenringförmig ausgebildet ist und aus einem in Bezug auf den Profilquerschnitt des Schlemmschen Kanals (5) verformbaren und biolytisch abbaubarem Material hergestellt ist.

11. Vorrichtung nach Anspruch 10, **dadurch gekennzeichnet,** dass zur Vermeidung einer Kippbewegung des implantierten Stützelements (30) dessen Breite (31) grösser als die Höhe des im Profilquerschnitt etwa ellipsenförmigen Schlemmschen Kanals (5) ausgebildet ist.

12. Vorrichtung nach Anspruch 9, **dadurch gekennzeichnet,** dass das Stützelement (33) als Kugel ausgebildet ist und mindestens eine Durchgangsbohrung (34), vorzugsweise eine Vielzahl in Umfangsrichtung verteilt zueinander angeordnete Durchgangsbohrungen (34) aufweist und aus biolytisch abbaubarem Material hergestellt ist.

13. Vorrichtung nach Anspruch 9, **dadurch gekennzeichnet,** dass das mit einer Durchgangsbohrung (36) versehene Stützelement (35) als ein im Profilquerschnitt kreisring- oder ellipsenringförmiges und mit einer Anzahl im Abstand zueinander angeordneter Öffnungen (37) versehenes Röhrchen ausgebildet ist, welches in Bezug auf die theoretische Längsachse (X) hinsichtlich der Lage und Orientierung flexibel sowie frei beweglich ausgebildet und aus biolytisch abbaubarem Material hergestellt ist.

14. Vorrichtung nach Anspruch 9, **dadurch gekennzeichnet,** dass das mit einer Durchgangsbohrung (41') versehene Stützelement (40) als ellipsenförmiges Röhrchen ausgebildet ist und an der Breitseite eine Anzahl in axialer Richtung im Abstand zueinander angeordnete Öffnungen (42,42') und mindestens zwei in axialer Richtung im Abstand zueinander angeordnete sowie an der Schmalseite radial nach aussen abstehend angeformte Auflageteilten (43,43',43") aufweist.

15. Vorrichtung nach Anspruch 14, **dadurch gekennzeichnet,** dass das mit den angeformten Auflageteilten (43,43',43") versehene Stützelement (40) aus biolytisch abbaubarem Material hergestellt ist.

16. Vorrichtung nach Anspruch 9, **dadurch gekennzeichnet,** dass das längliche Stützelement (45) als flexibles sowie hinsichtlich der Lage und Orientierung frei bewegliches Netzgeflecht ausgebildet ist.

17. Vorrichtung nach Anspruch 16, **dadurch gekennzeichnet,** dass das längliche Stützelement (45) aus biolytisch abbaubarem Material hergestellt ist.

18. Vorrichtung nach Anspruch 9, **dadurch gekennzeichnet,** dass das längliche Stützelement (50) in Form einer flexiblen Schraubenfeder ausgebildet sowie hinsichtliche der Lage und Orientierung frei beweglich ist.

19. Vorrichtung nach Anspruch 18, **dadurch gekennzeichnet,** dass das längliche Stützelement (50) aus biolytisch abbaubarem Material hergestellt ist.

20. Vorrichtung nach Anspruch 9, **dadurch gekennzeichnet,** dass das Stützelement (30;33;35;45;50) aus einem innerhalb eine Zeitdauer von 2 bis 12 Monaten nach der Implantation von dem Gewebe des Schlemmschen Kanals (5) und/oder von dem Kammerwasser biolytisch abbaubarem Material besteht.

21. Vorrichtung nach Anspruch 20, **dadurch gekennzeichnet,** dass das einzelne Stützelement (30;33;35; 45;50) aus vernetztem Natriumhyaluronat hergestellt ist.
